# EUROPEAN PATENT APPLICATION

(11) **EP 4 227 958 A1**
(43) Date of publication of application: **16.08.2023**
(21) Application number: 23153335.7
(22) Date of filing: 25.01.2023
(51) Int. Cl.: G16H 50/20

(54) **METHOD AND SYSTEM FOR RECOMMENDING INJECTABLES FOR COSMETIC TREATMENTS**

(30) Priority: 11.02.2022 US 202263267886 P
(71) Applicant: 13518221 Canada Inc., Calgary AB T2T 0K6 (CA)
(72) Inventor: DELAET, Sarah, Calgary, T2T 0K6 (CA); CURNEW, George, Calgary, T2T 0K6 (CA); MILLOY, Kyle, Calgary, T2T 0K6 (CA); YU, Ron, Calgary, T2T 0K6 (CA)
(74) Representative: FRKelly

(57) **Abstract**

The present disclosure provides a system and method for recommending injectables for cosmetic treatments. An input image including a body regions such as face, is received. The system uses a machine learning module to detect one or more injectable zones within the body region. The system determines an aesthetic score of the body region based on the injectable zones and identifies one or more injectable zones that can be modified by injecting injectables to achieve an augmented body region that has a revised aesthetic score satisfying a predefined threshold. The system then generates an output recommendation image to be displayed on an output device. The output recommendation image indicates the system identified one or more injectable zones that can be modified.

## Description

### CROSS REFERENCE TO PRIOR APPLICATIONS

The present application claims priority from U.S. Patent Application No. 63/267,886, filed on February 11, 2022, the contents of which are incorporated herein by reference.

### TECHNICAL FIELD

The present disclosure generally relates to cosmetic procedures, and more particularly, relates to a system and method for recommending injectables, such as facial injectables, for such cosmetic procedures.

### BACKGROUND

Cosmetic treatments, including surgical and non-surgical procedures, have gained immense popularity over the years. More particularly, non-surgical treatments, such as facial injectables, have been highly popular among older as well as younger population for enhancing their attractiveness. Consequently, cosmetic treatments have become a sought-after career option for many. For instance, there are many professionals who practice, especially in the area of non-surgical cosmetic enhancements such as injectables. Nevertheless, similar to any other medical procedure, cosmetic treatments also pose risks of potential complications that may be caused by even the slightest of errors. Although many risks associated with cosmetic treatments are generally temporary, in some rare cases, they may even contribute to more permanent damages. Therefore, performing such treatments is considered a highly intricate job and requires extensive training and experience.

Further, while the importance of accurate treatment is extremely high, the treatment itself comes into picture at a later stage and often patients need to make up their mind before actually going through the process. Conventionally, patients discussed these treatments with the professional who explained, often verbally, how the particular treatment may look like on the patient. Lately, many Augmented Reality (AR) based visualization applications have been developed that facilitate visualization of how a particular body part, such as face, will look like with augmentations and enhancements. However, not only can such visualizations not be replicated in real life, but these AR applications do not provide any assistance to the professional in performing the treatment whatsoever.

Therefore, there exists a need for a system that not only facilitates effective visualizations of treatments for the patients, but can also accurately assist professionals in carrying out such cosmetic procedures.

### SUMMARY

In one aspect of the present disclosure, a method for recommending injectables for a cosmetic treatment is provided. The method includes receiving, by a recommendation system processor, an input image including a body region of a user. The method further includes detecting, by the recommendation system processor including a machine learning module, one or more injectable zones within the body region. The method then determines an aesthetic score of the body region based on the detected one or more injectable zones. Further, the method identifies at least one injectable zone to be modified by injecting an injectable for achieving an augmented body region having a revised aesthetic score that satisfies a predefined threshold. Finally, the method includes generating an output recommendation image to be displayed on an output device associated with the one or more user devices. The output recommendation image indicates the identified at least one injectable zone that can be modified.

In another aspect of the present disclosure, a system for recommending injectables for a cosmetic treatment is provided. The system includes an input/output unit for receiving one or more inputs from and providing output to one or more user devices, a memory unit, and a recommendation system processor operative coupled to the input/output unit and the memory unit. The recommendation system processor receives an input image that includes a body region of user via a user interface displayed on the user device. The processor is configured to use a machine learning module to detect one or more injectable zones within the body region in the input image. Further, the processor determines an aesthetic score of the body region based on the detected one or more injectable zones. The system then identifies at least one injectable zone that can be modified by injecting an injectable for achieving an augmented body region that has a revised aesthetic score satisfying a predefined threshold. The processor further generates an output recommendation image to be displayed on an output device associated with the one or more user devices. The output recommendation image indicates the identified at least one injectable zone that can be modified.

### BRIEF DESCRIPTION OF THE DRAWINGS

Embodiments will now be described with reference to the appended drawings wherein:
FIG. 1 illustrates a computing environment including an example of an injectables recommendation system, in accordance with the embodiments of the present disclosure;
FIG. 2 illustrates an example recommendation system processor of the injectables recommendation system, in accordance with the embodiments of the present disclosure;
FIGS. 3 and 4 illustrate examples of graphical user interfaces (GUIs) displayed on a user device for training a machine learning module of the injectables recommendation system, in accordance with the embodiments of the present disclosure;
FIG. 5 illustrates an example method of providing recommendations for injectables, in accordance with the embodiments of the present disclosure;
FIGS. 6 and 7 illustrate examples of graphical user interfaces (GUIs) displayed on a patient's device, in accordance with the embodiments of the present disclosure;
FIGS. 8 and 9 illustrate an exemplary graphical user interface (GUI) displayed on a practitioner's device, in accordance with the embodiments of the present disclosure; and
FIG. 10 illustrates an example method for recommending injectables using machine learning, in accordance with the embodiments of the present disclosure.

### DETAILED DESCRIPTION

At the outset, it will be appreciated that like drawing numbers on different drawing views identify identical, or functionally similar, structural elements of the described system. It will also be appreciated that figure proportions and angles are not always to scale in order to clearly portray the attributes of the present disclosure.

Cosmetic treatments, if done incorrectly, can pose risks of potential complications that may range from minor temporary issues to more significant or even permanent damages. An important aspect to take care of while planning such cosmetic treatments is that the specific locations for such treatments, e.g., locations for injecting facial injectables, must be accurately identified. Even a slight misidentification of such locations may result in a significant damage, which is obviously not desired. Additionally, the types of injectables and/or the amount of injectables to be used for a particular treatment are also very important to ensure the accuracy and effectiveness of the treatment and minimize the potential risks.

For instance, in cases of facial cosmetic enhancements, neuromodulator injectables, such as botulinum toxin (commonly known as Botox), may pose minor or temporary potential complications, such as a droopy eyelid, facial asymmetry, or the like, which are generally reversible and may diminish within a few weeks or months. However, on the other hand, certain soft tissue or derma fillers, such as Hyaluronic Acid fillers, Calcium Hydroxylapatite fillers, and the like, if injected through a blood vessel accidently, may not only lead to temporary complications, e.g., change in color or death of a tissue in the treated area, but may also pose a relatively higher risk of permanent damage, such as blindness, or even stroke in some rare cases. Therefore, it is very important that the practitioners or professionals performing such treatments are experienced and highly trained in the domain.

Furthermore, because these cosmetic enhancements are not easily reversible and/or are very expensive to reverse, it is desirable to have a visualization of the cosmetic treatment for the patients and the professionals before actually going through the procedure. Such visualizations not only help the patient in visualizing how they may look after the treatment and decide if they really want to go through with it, but also allow the patients and/or the practitioners to make any adjustments and customizations to the treatment to make it more suited to their liking.

To this end, the present disclosure provides a system and method for recommending injectables in cosmetic procedures. The system may be configured to accurately identify locations, types and/or quantities for injectables to be used by a practitioner, e.g., a cosmetic surgery professional, in performing the cosmetic treatment. The system may further be configured to provide visualizations of 'before' and `after' treatment results of the cosmetic treatment and/or the recommendations provided by the system for such treatment. As will be explained later, the embodiments described herein not only provide accurate recommendations, and effective visualizations, but also make the entire treatment efficient for the practitioner and enhances the reliability and security of the treatment for a patient.

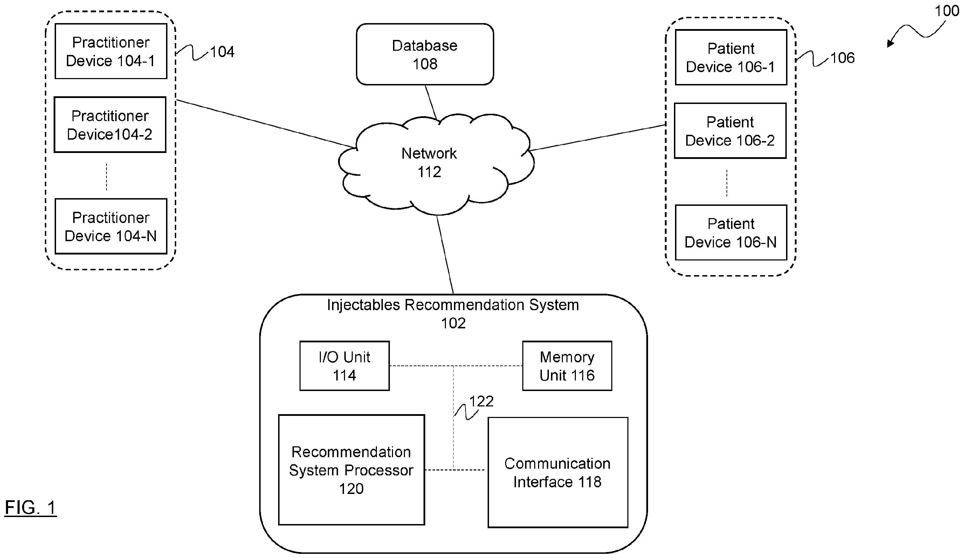

Fig. 1 illustrates an example of a computing environment 100 including an injectables recommendation system 102, according to the embodiments of the present disclosure. The present description is provided for facial injectables, including but not limited to, neuromodulator injectables, soft tissue injectables, derma fillers, and the like. However, it will be appreciated that the injectables recommendation system 102 may additionally or alternatively be implemented for recommending injectables for any other body region in a similar manner without deviating from the scope of the claimed subject matter. According to an embodiment of the present disclosure, the injectables recommendation system 102 (hereinafter referred to as the recommendation system 102) is configured to provide recommendations using machine learning for cosmetic treatments, such as the facial injectables. For example, the recommendation system 102 may automatically make recommendations, such as locations for injectables, type of injectables, and/or quantity of injectables, and provide a visualization of the treatment results corresponding to the various treatment options provided or recommended by the recommendation system 102. In some implementations, the recommendation system 102 may also provide a customizable visualization for a user to manipulate and identify a preferred look and accordingly identify, recommend, and visualize the treatment options, for example, for the professional to perform the actual cosmetic treatment.

In an embodiment, the computing environment 100 (hereinafter referred to as the environment 100) may further include one or more first user devices 104 (e.g., practitioner devices 104-1, 104-2... 104-n), one or more second user devices 106 (e.g., patient devices 106-1, 106-2... 106-n), and a database 108, each communicating with one another and the recommendation system 102 via a network 112. Examples of the network 112 may include, but not limited to, a wide area network (WAN) (e.g., a transport control protocol/internet protocol (TCP/IP) based network), a cellular network, or a local area network (LAN) employing any of a variety of communications protocols as is well known in the art. In some embodiments, the environment 100 may alternatively be implemented in a cloud-based computing environment.

Each practitioner device 104 provides an interface for a respective professional or practitioner interacting with the recommendation system 102, whereas each patient device 106 provides an interface for a respective patient or potential patient interacting with the recommendation system 102. Examples of a practitioner or a professional may include, but not limited to, a plastic surgeon, dermatologist, facial plastic surgeon, oculoplastic surgeon, general physician, nurse, dentist, dental surgeon, or the like, who practice in the field of such cosmetic treatments for which the recommendation system 102 is implemented. In an example, each of the user devices 104, 106 may be embodied as one of a personal computer, desktop computer, tablet, smartphone, or any other computing device capable of communicating with the recommendation system 102. Each of the user devices 104, 106 may include appropriate interface(s), such as a display screen, touch screen, keyboard, or any other input/output device, to facilitate providing inputs to and receiving output from the recommendation system 102. Each user (i.e., the practitioners and the patients) may utilize the respective user devices 104, 106 to provide one or more user inputs and receive one or more outputs, for example, from the recommendation system 102. In some embodiments, the one or more user devices 104, 106 may include an application (such as a mobile application) or a web portal or any other suitable interface running thereon and hosted by the recommendation system 102, through which the respective user may communicate and interact with the recommendation system 102. In some embodiments, each user device 104, 106 may include a plurality of electrical and electronic components, providing power, operational control, communication, and the like. For example, each user device 104, 106 may include, among other things, its own transceiver, display device, network interface, processor, and a memory (not shown) that cooperate to enable operations of the corresponding user device 104, 106. Such components of the user devices 104, 106 are commonly known and hence not described herein in greater detail for the sake of brevity of the present disclosure.

The database 108 may be configured to store the one or more documents, images, records, and/or any other data associated with and/or generated by the recommendation system 102. The database 108 may be queried by the recommendation system 102 to retrieve relevant information corresponding to or in response to one or more queries received from the one or more user devices 104, 106. For example, the database 108 may be an internal and/or an external database and may be implemented using relational databases, such as, but not limited to, Sybase, Oracle, CodeBase, and Microsoft^{®} SQL Server or other types of databases such as, a flat file database, an entity-relationship database, an object-oriented database, a record-based database, or any other type of database known presently or may be developed in the future. It will be appreciated that the database 108 may include any volatile memory elements (e.g., random access memory (RAM), nonvolatile memory elements (e.g., ROM), and combinations thereof. The database 108 may also incorporate electronic, magnetic, optical, and/or other types of storage media.

As illustrated, in an example embodiment of the present disclosure, the recommendation system 102 includes an input/output (I/O) unit 114, a memory unit 116, a communication interface 118, and a recommendation system processor 120. It will be appreciated by those of ordinary skill in the art that Fig. 1 depicts the recommendation system 102 in a simplified manner and a practical embodiment may include additional components and suitably configured logic to support known or conventional operating features that are not described in detail herein. It will further be appreciated by those of ordinary skill in the art that the recommendation system 102 may be implemented as a server, a personal computer, a desktop computer, a tablet, a smartphone, or as any other computing device known now or that may be developed in the future.

Further, although the entire recommendation system 102 is shown and described to be implemented within a single computing device, it may be contemplated that the one or more components of the recommendation system 102 may alternatively be implemented in a distributed computing environment, without deviating from the scope of the claimed subject matter. It will further be appreciated by those of ordinary skill in the art that the recommendation system 102 alternatively may function within a remote server, a cloud computing device, or any other remote computing mechanism known presently or may be developed in the future. For example, the recommendation system 102, in some embodiments, may be a cloud environment incorporating the operations of the I/O unit 114, the memory unit 116, the communication interface 118, the recommendation system processor 120, and various other operating modules to provide the functionalities provided herein this disclosure.

The components of the recommendation system 102, including the input/output unit 114, the memory unit 116, the communication interface 118, and the recommendation system processor 120, may communicate with one another via a local interface 122. The local interface 122 may include, but not be limited to, one or more buses or other wired or wireless connections, as is known in the art. The local interface 122 may have additional elements, which are omitted for simplicity, such as controllers, buffers (caches), drivers, repeaters, and receivers, among many others, to enable communications. Further, the local interface 122 may include address, control, and/or data connections to enable appropriate communications among the aforementioned components.

The I/O unit 114 may be used to receive one or more inputs from and/or to provide one or more system outputs to one or more devices or components. For example, the I/O unit 114 may be configured to receive one or more inputs from the practitioners and/or the patients, as will be described later herein, and provide output to the one or more users, such as those of the practitioner devices 104 and patient devices 106 interacting with the recommendation system 102. System input may be received by the I/O unit 114 via, for example, a keyboard, touch screen, touchpad, mouse or any other input device associated with the recommendation system 102 and/or the user devices 104, 106. System output may be provided by the I/O unit 114 via, for example, a display device, speakers, printer (not shown) or any other output device associated with the recommendation system 102 and/or the user devices 104, 106.

The memory unit 116 may include any of the volatile memory elements (e.g., random access memory (RAM), nonvolatile memory elements (e.g., ROM), and combinations thereof. Further, the memory unit 116 may incorporate electronic, magnetic, optical, and/or other types of storage media. It may be contemplated that the memory unit 116 may have a distributed architecture, where various components are situated remotely from one another, and are accessed by the recommendation system 102, and its components, such as the recommendation system processor 120. The memory unit 116 may include one or more software programs, each of which includes listing of computer executable instructions for implementing logical functions. The software in the memory unit 116 may include a suitable operating system and one or more programming codes for execution by the components, such as the recommendation system processor 120 of the recommendation system 102. The operating system may be configured to control the execution of the programming codes and provide scheduling, input-output control, file and data management, memory management, and communication control, and related services. The programming codes may be configured to implement the various processes, algorithms, methods, techniques, etc. described herein.

The communication interface 118 may be configured to enable the recommendation system 102 to communicate on a network, such as the network 112, a wireless access network (WAN), a radio frequency (RF) network, and the like. The communication interface 118 may include, for example, an Ethernet card or adapter or a wireless local area network (WLAN) card or adapter. Additionally, or alternatively, the communication interface 118 may include a radio frequency interface for wide area communications such as Long-Term Evolution (LTE) networks, or any other networks known now or developed in the future. The communication interface 118 may include address, control, and/or data connections to enable appropriate communications on the network 112.

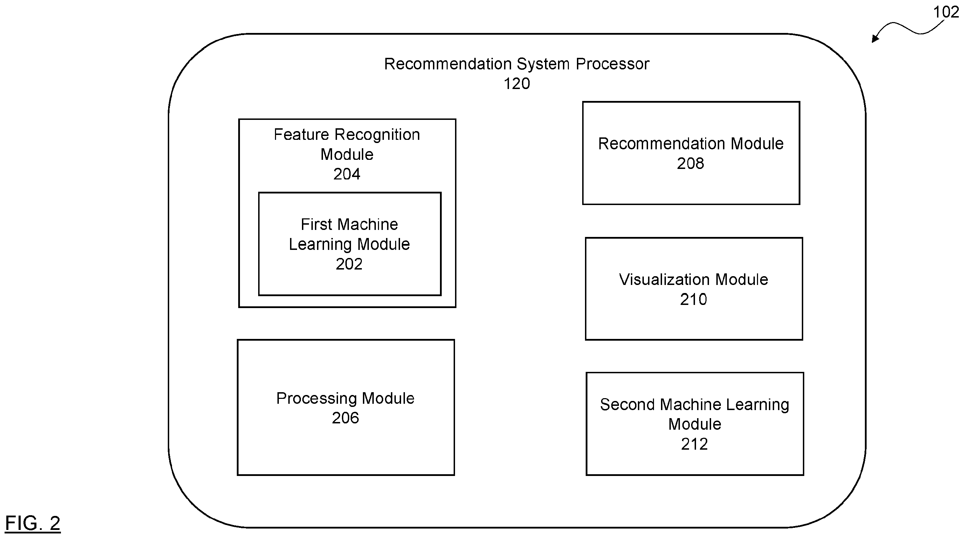

The recommendation system processor 120 may be a hardware device for executing software instructions, such as the software instructions stored in the memory unit 116. The recommendation system processor 120 may include one or more of a custom made or commercially available processor, a central processing unit (CPU), an auxiliary processor among several processors associated with the recommendation system processor 120, a semiconductor-based microprocessor, or generally any device for executing software instructions. When the recommendation system 102 is in operation, the recommendation system processor 120 may be configured to execute software stored within the memory unit 116 to generally control and perform the one or more operations of the recommendation system 102 pursuant to the software instructions. The details of the recommendation system processor 120 will now be described in greater detail with reference to Figs. 2 through FIG. 10.

Referring now to Fig. 2, the recommendation system processor 120 includes one or more components cooperating with one another to provide recommendations for injectables. As shown, the recommendation system processor 120 includes a feature recognition module 204, a first machine learning module 202, a processing module 206, a recommendation module 208, and a visualization module 210. In some implementations, the recommendation system processor 120 further includes a second machine learning module 212. Detailed functioning of these modules will now be described.

As the recommendation system processor 120 receives one or more images containing a body region, the image is processed to detect one or more injectable zones and make recommendations on which injectable zones could be injected to achieve an enhanced or augmented body region. In an embodiment of the present disclosure, the feature recognition module 204 incorporates or uses the first machine learning module 202 to predict or identify one or more injectable zones within a particular body region, such as the face of a user. The injectable zones may include, but are not limited to, one or more zones within the body region that may be capable of receiving injectables. The first machine learning module 202 may be configured to use supervised or unsupervised learning to predict and/or identify one or more injectable locations within any body region. In one example, the first machine learning module 202 may utilize a deep learning framework, such as Residual Network (ResNet) Convolutional Neural Network (CNN) and/or Modular Neural Network (MNN), for training. The first machine learning module 202 may be configured to be trained using a number of training images received, for example, from one or more of practitioner devices 104, to learn to process unmarked images and identify one or more injectable zones and landmark physical features within the body region present in the unmarked images. For example, training data including a number of images having human faces along with a number of associated information is provided to the first machine learning module 202. The training data may include images with predefined injectable zones, such as facial zones, that are represented as location coordinates along a lateral axis, i.e., x-axis and a longitudinal axis, i.e., y-axis of a face. Facial zones correspond to the injectable zones in the face that are capable of being injected with an injectable to create an end augmented result for the face. Examples of the facial zones may include, but not limited to, under eye areas, cheeks, lips, upper lip region, sides of chin, center of chin, jaw lines, hairline, eyelids, forehead, or any other areas in the face that may be capable of receiving the facial injectables. In some examples, the injectable zones may correspond to specific injection points in the body region and the associated information with these injectable zones may include the name and type of the injection point being marked.

In one example, for a given set of training images, 70% of the images with marked or defined injectable zones are provided for training the first machine learning module 202. The training data may be provided by one or more highly trained and experienced practitioners, such as cosmetic or plastic surgeons, physicians and/or nurses, practicing in a domain in which the system is implemented. For example, the training data may be received from these practitioners via their respective practitioner devices 104 by the I/O unit 114 of the recommendation system 102 over the network 112. In some other implementations, the database 108 may store a number of images along with the associated information (for example, as provided by the practitioners) which may be used as training data for the first machine learning module 202. In some implementations, the training data may additionally include variations to one or more images, such as translated or rotated images along with the accordingly adjusted coordinates of the identified injectable zones. Such variations facilitate enlargement of the training data and provides more variables for the first machine learning module 202 to learn, for example, to identify the injectable zones corresponding to the yaw, pitch, and tilt of the face.

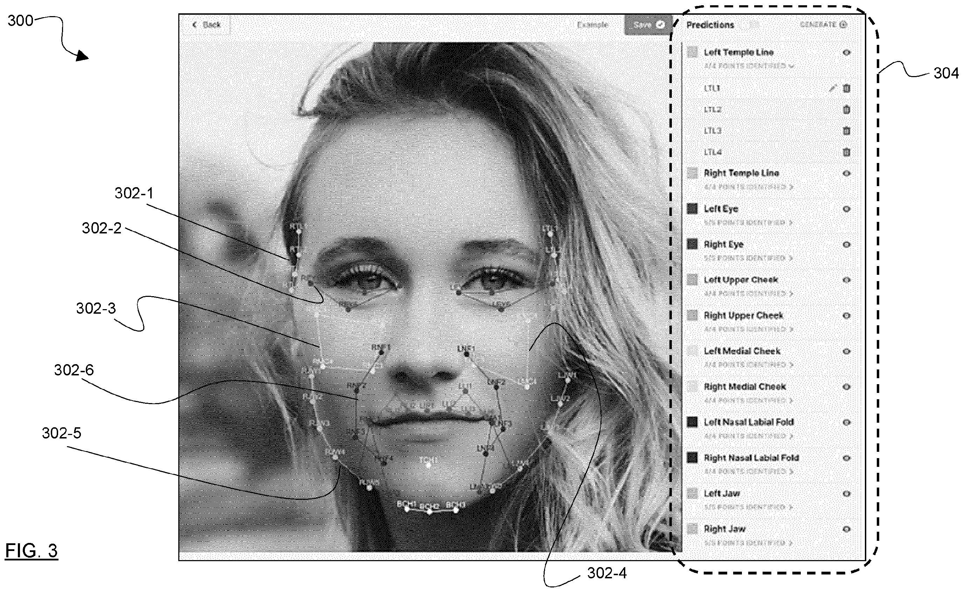

Fig. 3 illustrates an example graphical user interface (GUI) 300 that may be displayed, for example, on a display associated with the practitioner's device 104 to facilitate inputting marked images to the first machine learning module 202 for training. As illustrated, the practitioner may upload an image and mark (e.g., by using a mouse or touch-enabled input unit of the device 104) a number of injectable zones, e.g., right temple line 302-1, right eye 302-2, right medial cheek 302-3, left medial cheek 302-4, right jaw line 302-5, right nasal labial fold 302-6, and so on (only a few injectable zones are marked and shown in Fig. 3) in the face present in the image. As the practitioner selects these zones on the face, the location (x/y) coordinates of the marked zone are automatically extracted by the module 202, for example, by using deep learning or computer vision or other image processing techniques. The practitioner may mark the injectable zones, including but not limited to, temple lines, eyes (corresponding to the left and right under eyes), upper cheeks, medial cheeks, nasal labial folds, jaws, and so on and adjust their coordinates by manipulating them through a number of user input options provided on the interface 300, such as that shown in section 304. It may be appreciated that the names corresponding to these injectable zones may be predefined in the system 102 or they may be provided by the practitioner while training the system. Further, these names are merely examples and may be varied without deviating from the scope of the present disclosure. Similarly, the practitioners may upload other images (typically hundreds or thousands of images) with marked injectable zones in a similar manner and all these marked images are then stored in the database 108 for further training the first machine learning module 202. Further, at the system end, the first machine learning module 202 is configured to receive these images along with the predefined location (x/y) coordinates of injectable zones as marked by the practitioner(s) (hereinafter referred to as 'real coordinates' of the injectable zones) and learn to detect the injectable zones by identifying and correlating patterns, for example, between the coordinates of the injectable zones with respect to the coordinates of other landmark features of the face, such as nose, lips, eyes, ears, and the like.

In some implementations, the training data for the first machine learning module 202 may also include types of injectables, such as Hyaluronic Acid injectables, Botox injectables, and so on, (e.g., that may also be classified by their brand name and their corresponding viscosity and elasticity) that may be suitable for each of the marked injectable zones. Further, the training data may also include one or more injection planes for injectables, i.e., in which plane, a particular injectable can be injected. In such implementations, the first machine learning module 202 may receive inputs from the practitioner or additionally from third party or auxiliary data sources, such as guidelines provided by health authorities, for such injectables. To this end, the interface 300 may also allow the practitioner to mark the injection planes and enter the types and quantity of the injectables corresponding to the various injectable zones and planes, in a similar manner as described above. In some implementations, the training data may also include facial structure definitions (e.g., bone structure, facial landmarks such as nose, lips, eyebrows, eyes, tissue volume in upper third, middle-third, and/or lower third of the face), along with any other information that may be useful for the first machine learning module 202 in learning to identify the injectable zones and landmark features in any patient's face. Such facial structure definitions may be identified or extracted by a processing module 206 by using one or more of deep learning, computer vision or image processing tools, which are then used by the first machine learning module 202 to also learn to detect and identify these facial features on unmarked images. In some other implementations, the processing module 206 may also communicate with one or more additional resources, such as web pages, credible online knowledge portals, and/or the practitioners to receive auxiliary information associated with injectables to be used as input along with the training data to the first machine learning module 202. Examples of the auxiliary information may include, but not limited to, potentially dangerous zones that are not suitable for injectables, recommended quantity of injectables corresponding to their types, and so on.

Further, a test data including a number of test images (in this example, remaining 30% of the training images with hidden marked or defined injectable zones) may be provided to the first machine learning module 202 for automatically identifying the injectable zones. The test data may include test images with hidden injectable zones and the first machine learning module 202 may be configured to identify or predict the coordinates of the injectable zones for these images. The test data may also include translated or rotated images to test the identification capabilities of the first machine learning module 202. The generated results from the first machine learning module 202 may be validated against the predefined injectable zones marked for the corresponding image in the test data. In one implementation, the first machine learning module 202 may be configured to output x/y coordinates (e.g., in the form of marked injectable zones or stream JavaScript Object Notation (JSON)-data containing the x/y coordinates and the like) of the predicted injectable zones in the image. The test data may also be stored in the database 108 along with the associated predefined injectable zones. Feedback regarding the accuracy of the identified injectable zones may be provided to retrain the first machine learning module 202 and enhance its accuracy.

Furthermore, the first machine learning module 202 may be configured to receive new images, for example, from one or more practitioners, new users or patients, and predict or detect one or more injectable zones in these images. In an example, the generated output (i.e., the detected injectable zones) may also be provided to the one or more practitioners, via the corresponding devices 104, for validation and their feedback may be used to further retrain the first machine learning module 202. The first machine learning module 202 continuously and iteratively learns to enhance the accuracy and fidelity of the predicted injectable zones and its overall capabilities.

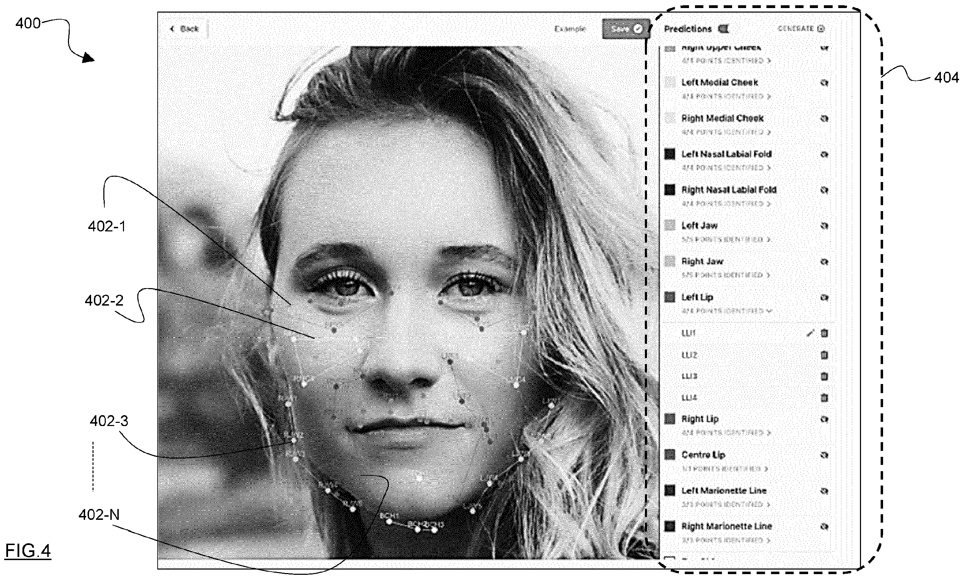

Referring now to Fig. 4, another example of a GUI 400 is illustrated. The GUI 400 may be displayed, for example, on the display associated with the practitioner's device 104 to facilitate validating the predicted output from the first machine learning module 202. As shown, as the practitioner sends or uploads an image on to the system 102, the first machine learning module 202 predicts and displays the predicted injectable zones and their coordinates 402. The system generated output coordinates of the injectable zones (hereinafter referred to as the "predicted coordinates"), e.g., the predicted injectable zones 402-1, 402-2, 402-3...402-N shown in FIG. 4, are then compared with the real coordinates that have been marked or provided for the same image by one or more practitioners either initially as part of the training data or as a feedback for new images. In an embodiment, the processing module 206 may be configured to compare the predicted coordinates with the real coordinates to determine the accuracy of the predictions output by the first machine learning module 202. Further, in some implementations, the processing module 206 may also facilitate the practitioner to adjust the coordinates and accordingly the positioning of the predicted injectable zones (e.g., via the section 404 of the interface 400). This may be done for one or more of the training images, test images or even the new images provided to test the first machine learning module 202 for retraining. These feedbacks are stored in the database 108 and may be used for further training of the first machine learning module 202 and to enhance its prediction capabilities.

Further, in some implementations, the first machine learning module 202 may also predict and display injection planes within the body region, i.e., the face in this example, along with types and quantities of the injectables on the interface 400. The generated output may then be compared by the processing module 206 with the real recommendations that have been provided for the same image by the practitioner(s), either initially as training data or as part of a feedback for new images, to determine the accuracy of the predictions output by the first machine learning module 202. In such implementations, the interface 400 may also allow the practitioner to adjust one or more of the predicted parameters, thereby providing the feedback to retrain the first machine learning module 202.

The usage of machine learning by the recommendation system 102 may result in more and more accurate predictions of injectable zones as well as other parameters like the injection plane, and type and quantity of injectables, over time that are less prone to human error and thus provide an enhanced overall safety and security for the injectables that are injected into the patients. The recommendations generated by the recommendation system 102 may then be used by the practitioners and the patients to efficiently and accurately plan and perform the final cosmetic treatment.

In operation, the recommendation system processor 120 may receive an image with a patient's body region from one or more patient device 106 or one or more practitioner device 104 via the I/O unit 114 over the network 112. As explained above, the user devices 104, 106 may include an interface, e.g., a mobile application or a web application, which facilitates the corresponding patient or practitioner to provide the image(s) to the recommendation system 102 and request recommendations for one or more cosmetic enhancements. The feature recognition module 204 may be configured to utilize the first machine learning module 202 to process the image and detect one or more injectable zones, their location (x/y) coordinates, the injection planes, and type and quantity of injectables for enhancing the attractiveness of the patient body region.

In an embodiment, once the location coordinates of the injectable zones are detected, the processing module 206 may be configured to determine one or more feature ratios for the body region based on the detected location coordinates of each of identified injectable zones. For instance, in the case of a human face, examples of feature ratios may include, but not limited to, a ratio of length of the face to the width of the face, or ratio of a distance between landmark physical features, such as nose, eye, cheeks, chin, and the like. The processing module 206 may further be configured to determine an aesthetic or attractiveness score of the body region based on the determined feature ratios. For example, the processing module 206 may be configured to use the detected injectable zones, their location coordinates, and the corresponding feature ratios to perform one or more analyses of the body region against a predefined criteria, such as the 'Golden Ratio' criteria, to determine the aesthetic score for the body region. The aesthetic score may be indicative of a degree of match between the feature ratios of the body region and the predefined criteria, in this example, the golden ratio. As will be appreciated, the golden ratio for a body region, such as the face, may be defined as an ideal ratio for symmetry that makes the body region ideally attractive. For example, in the case of a face, the golden ratio provides that a feature ratio of the length of the face (i.e., from the top of the head to the chin) to the width of the face (i.e., from ear to ear), may ideally be 1:1.618. In another example, a feature ratio of a distance from top of the nose to the center of the lips and a distance between the center of the lips and the chin should be 1:1.6. Similarly, a feature ratio of the distance from hairline to the upper eyelids with the length of the top of the upper eyebrows to the lower eyelids must be 1:1.6. In a yet another example, the ideal ratio of upper to lower lip volume is 1:1.6. These and other similar feature ratios may be used individually or together to identify the attractiveness of the patient's body region and determine the aesthetic score.

In an embodiment, the processing module 206 is configured to compare the determined feature ratios of the body region with the golden ratio to determine the aesthetic score indicative of how close or far the determined feature ratios are to the golden ratio. The aesthetic score may be represented in any suitable manner or format, such as a percentage score (e.g., the feature ratio may be a 75% close to the golden ratio). This means that a low aesthetic score, e.g., a score of 40%, indicates that the feature ratios are far from the golden ratio and a high aesthetic score, e.g., a score of 80%, indicates that the feature ratios are close to the golden ratio. In some examples, the scores and how they map on the scale of attractiveness with respect to the predefined criteria, i.e., the golden ratio in this example, may be predefined, for example, by the practitioners or may be set as part of an industry standard. It may be appreciated that the golden ratio criteria is merely an example and in some alternative implementations, other types of criteria may also be used to obtain the aesthetic score, without deviating from the scope of the claimed subject matter.

Generally, due to many reasons, such as age among others, certain facial measurements may tend to deviate farther from the golden ratio, thus reducing the attractiveness of the body region. For instance, fat around eyes, cheekbones, inner jawline, and sides may disappear with age causing a face to lose volume, leaving patients with a sunken appearance, and thus disturbing the feature ratios of the face and leaving them more deviant from the golden ratio.

To this end, in an embodiment of the present disclosure, the recommendation system 102 is configured to determine the one or more injectable zones within the body region that could be injected with injectables to enhance the feature ratios and have a revised aesthetic score that is closer to the golden ratio, thereby making the body region more attractive. In one implementation, the processing module 206 may be configured to adjust the location coordinates of the one or more injectable zones, wherein the adjustments correspond to the modification that needs to be made by injecting the injectables. For example, a higher adjustment of location coordinates may indicate to the practitioner that a higher quantity or a specific type of injectable needs to be injected in order to achieve the modified injectable zone whereas a lower adjustment may indicate a lower quantity or some other type of injectables to be injected to achieve the modified injectable zone. The processing module 206 may further be configured to determine revised feature ratios for the body region corresponding to the adjusted location coordinates of the injectable zones and detect if the revised feature ratios result in an aesthetic score (hereinafter the "revised aesthetic score") that is closer to the golden ratio. Adjusting the location coordinates and evaluating the feature ratios and the revised aesthetic score(s) may, in some implementations, be an iterative process until the processing module 206 identifies one or more injectable zones that are most suitable for modification to achieve feature ratios that result in the revised aesthetic score closest to the golden ratio. For instance, the processing module 206 may determine that by augmenting the under eyes and smile lines, the patient's face may get closer to the golden ratio. In another example, the processing module 206 may be configured to determine that in order to reach the golden ratio, the forehead, the eye lids, under eyes, nose, cheeks, jaw lines, lips, and others may need to be augmented. Whereas in some yet other examples, the processing module 206 may determine that only cheeks need manipulation to achieve the golden ratio on the patient's face.

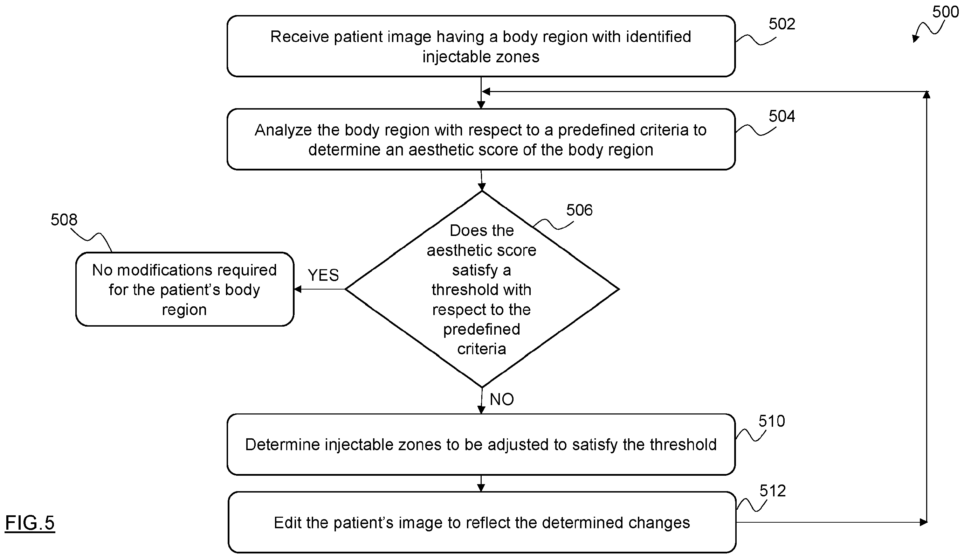

Referring now to Fig. 5, an example process 500 implemented by the recommendation system processor 120 to determine which injectable zones may require modification to achieve the aesthetic score closer to the golden ratio is illustrated. At step 502, a patient's image having a body region (e.g., the face), the identified injectable zones, and their location coordinates (i.e., detected by the first machine learning module 202) are received by the processing module 206. At step 504, the processing module 206 analyzes the body region with respect to the golden ratio, based on the feature ratios obtained from the location coordinates of the injectable zones, to determine the aesthetic score of the body region. If at step 506 it is determined the patient's body region has an aesthetic score that satisfies a predefined threshold with respect to the golden ratio (e.g., if they are 90% or more closer to the golden ratio), then the processing module 206 may determine that no modifications are required to the patient's body region and terminate the method at step 508. In such a case, the recommendation module 208, communicating with the processing module 206, may be configured to generate an alert to that effect for the patient and/or the practitioner, such as via a user interface displayed on the respective user device 104, 106.

However, if at step 506 it is determined that patient's feature ratios and the corresponding aesthetic score does not satisfy the threshold, then the processing module 206 proceeds to step 510. At step 510, the processing module 206 may be configured to determine the one or more injectable zones that may require augmentation or modifications by determining how the location coordinates of one or more of these injectable zones need to be displaced to get a more attractive face, i.e., as close to the golden ratio as possible. It may be appreciated that the threshold match of the patient's feature ratios with respect to the golden ratio may be dynamically defined, for example, by the practitioners, to suit every patient and may be defined to have a more natural enhanced look on the patient's face. For example, for one patient, the threshold may be set higher (such as up to 95%) to facilitate accommodating greater degree of enhancements without looking artificial or unnatural whereas for another patient, such threshold may be set lower (e.g., up to 70%) to ensure a lower degree of enhancements to the face without looking unnatural. In some other examples, the threshold may be preset to correspond to a range, such as 75% to 90% match with the golden ratio, to ensure that the patient's body region is only augmented to have a natural look.

Once the injectable zones that can be modified are determined, they are provided by the processing module 206 to the recommendation module 208, which further relays the adjusted location coordinates of the identified injectable zones to the visualization module 210. The visualization module 210, at step 512, may be configured to edit the received image accordingly to reflect the determined adjustments to one or more injectable zones and their coordinates. The visualization module 210 may be configured to use one or more of deep learning-based image adjustment tools, or any other image editing or video editing tools known in the art, to edit the received image. In an exemplary embodiment, the edited image is then re-checked to satisfy the threshold with respect to the golden ratio and is iteratively refined or edited until the threshold is satisfied, thereby terminating at step 508 where no further modifications are required to the image.

In some implementations, the recommendation system processor 120 may also facilitate a patient and/or the practitioner to visualize how a cosmetic treatment, or the augmented end result would look like on the patient. For example, one or more of an intermediate image (e.g., including the original location coordinates of the injectable zones while the initial image is being processed) and a final image having the final recommendations for modifiable injectable zones may be provided to the patient device 106 and/or the practitioner device 104. For example, the visualization module 210 may be configured to render the final recommended image (hereinafter referred to as the system recommended image) with the determined adjustments to be transmitted and displayed on a user interface displayed on one or more of the practitioner device 104 and the patient device 106. In an implementation, the visualization module 210 may be embodied as a three-dimensional (3D) visualization tool configured to generate visualizations in three dimensions. However, any other type of visualization tool, such as a two-dimensional (2D) visualization tool, may also be used instead, to achieve the desired results. In some implementations, instead of indicating the location coordinates in the image, the visualization module 210 may simply highlight the identified injectable zones in the system recommended image.

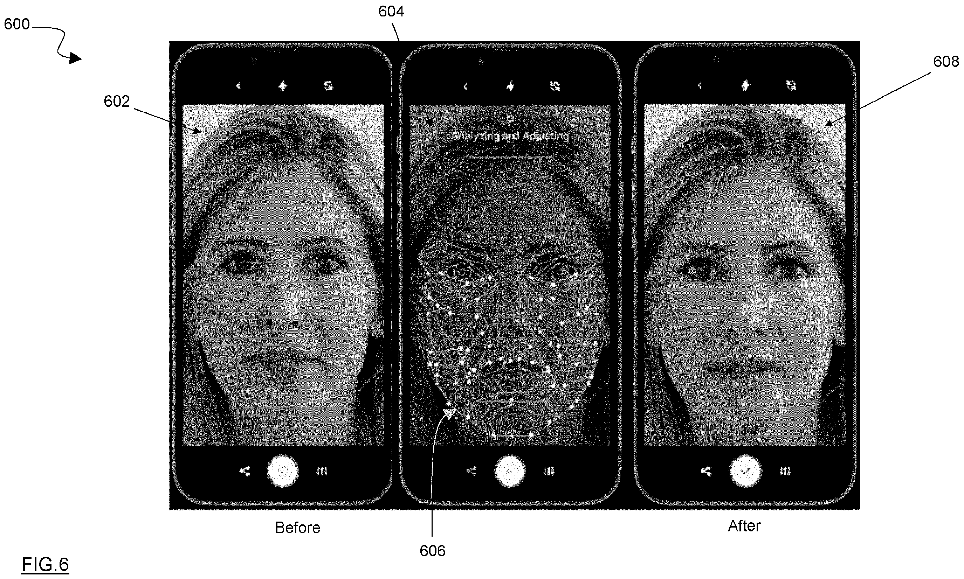

Fig. 6 illustrates an exemplary GUI 600 that may be displayed, for example, on a display associated with the practitioner device 104 and/or the patient device 106 to facilitate visualization of a "Before" and "After" effect according to the predicted output generated by the recommendation system 102 for one or more injectables. As illustrated, the interface 600 displays a "Before" image 602, an intermediate image 604 and an "After" image 608 for the patient and/or the practitioner to visualize how they would appear after the augmentations recommended by the recommendation system 102. For example, the intermediate image 604 may be displayed when the processing module 206 processes the received image 602 to determine the recommended injectable zones that can be modified by injecting injectables. In the illustrated embodiment, the displayed intermediate image 604 may include all the identified feature zones 606 and landmark features on the body region i.e., the face of the patient. Further, once the final image (corresponding to the adjusted coordinates of the injectable zones) is rendered by the visualization module 210, the same is displayed as the "After" image 608 (hereinafter referred to as the "system recommended image") with the face augmented in the recommended injectable zones. For example, as shown, the nasal labial folds, cheeks, under eyes, etc., on each side are shown as augmented in the "After" image 608 as compared to the initial "Before" image. It may be appreciated that such visualizations facilitate a patient to visualize their cosmetic enhancements before actually going through the process, which was not conventionally possible when interacting directly with the medical practitioner.

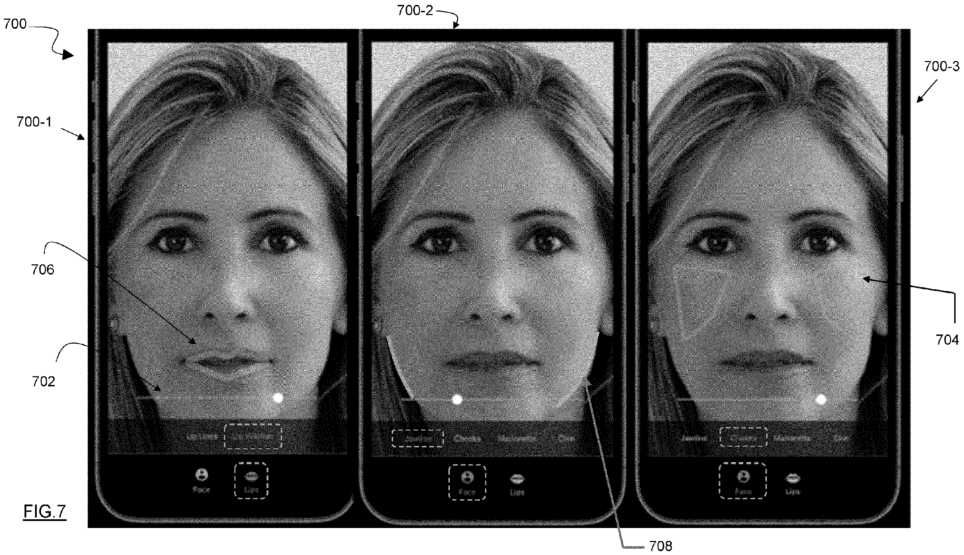

Further, in an embodiment of the present disclosure, the recommendation module 208 may further be configured to receive one or more user defined adjustments to the system recommended image, e.g., the "After" image 608 shown in FIG. 6. For example, as shown in Fig. 7, a GUI 700 displayed on the patient device 106 and/or the practitioner device 104 may include one or more adjustment tools 702 to allow them to add, remove or adjust the recommendations provided by the recommendation system 102. Thus, if the cheek enhancement recommended by the system 102 is not desired or liked by the patient, they may simply adjust (e.g., increase or decrease using a slider tool 702 to adjust the cheeks 704 shown in interface 700-3) or even remove this injectable zone adjustment. Further, the lip volume 706 (shown in interface 700-1) may also be adjusted by moving the slider tool 702. In another example, the jawlines 708 may also be adjusted by moving the slider tool 702 (as shown in interface 700-2). Similarly, in some examples, if the patient wants some additional enhancements that were not included in the system recommended image, they may do so by activating appropriate sections displayed on the interface. This way, the patients and/or the practitioners may be allowed to generate a "user customized image" by customizing and modifying the system recommended image to their liking. In an exemplary embodiment, as the patient moves the slider tool 702, the corresponding x/y coordinates of the respective injectable zones are adjusted by the processing module 206 at the backend, thereby adjusting the augmentation visible on the image displayed on the interface 700. Furthermore, in some additional embodiments of the present disclosure, the recommendation system 102 may also adjust the injection planes, types of fillers, quantity of fillers according to the adjustments to the coordinates of the injectable zones based on the user customized image. Further, in some implementations, the patient and/or the practitioner may be allowed to make adjustments to the image from different angles, such as for left profile, right profile and front profile, independent from one another, to allow the patient to ensure their customizations appear to their likings in all the profiles.

In various implementations of the present disclosure, one or more of the intermediary edited images, the system recommended images, user customized images and so on may be stored along with the patient's details in the database 108 for further training the recommendation system 102 and for future references by both the practitioners as well as the patients.

Further, the user customized image may be transmitted back to the processing module 206, wherein the processing module 206 may perform the golden ratio analysis on this received customized image in a similar manner as described above, to make any further recommendations or suggestions for patient's consideration and final approval. It may be appreciated that this system recommendation and user customization may be an iterative process in some implementations.

In case the customizations are provided by a patient, the user customized image received from the patient device 106 may be provided to a practitioner via the corresponding practitioner device 104. The practitioner may utilize the recommendations and the location coordinates of the injectable zones that are to be modified to generate a treatment regimen for the patient. For example, the visualization module 210 may transmit the final user customized image along with the identified injectable zones and recommendations for augmentation to be displayed on the corresponding practitioner device 104. In some embodiments, the initial image as provided by the patient may also be displayed on the practitioner device 104 to provide a 'before' appearance along with a tentative 'after' look corresponding to the user customized image.

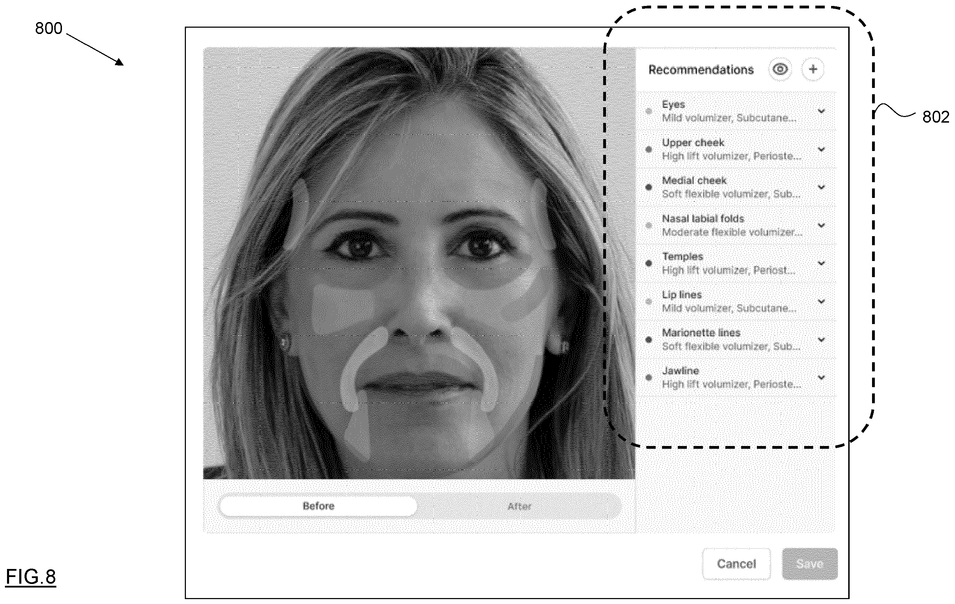

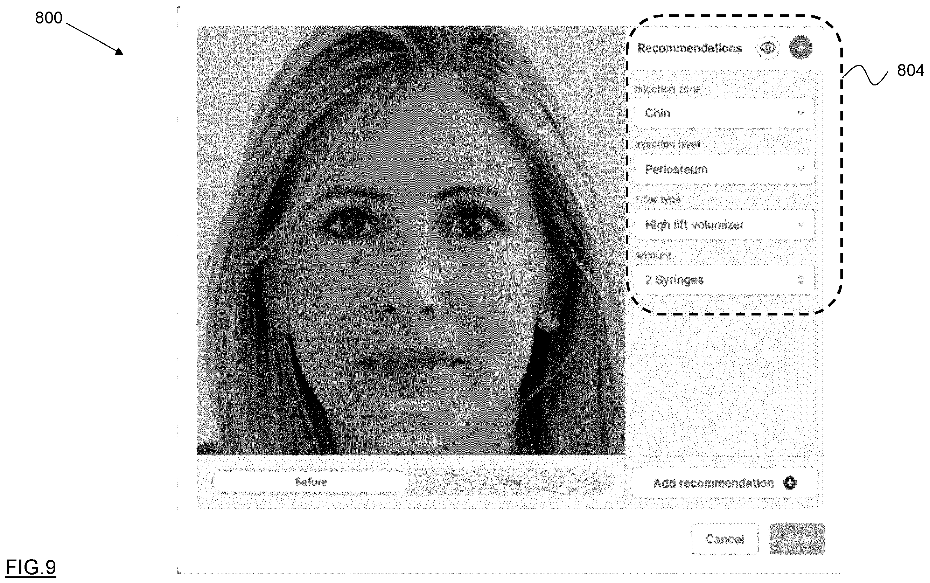

Further, the practitioner may define the amount, type, and location of the injectables to be injected based on the recommendations and locations of the injectable zones provided by the system 102 and as customized by the user. For example, as shown in Fig. 8, the user interface 800 that may be displayed on, for example, the practitioner device 104 may allow the practitioner to see the system recommendations, the customer adjustments and accordingly define the types and quantities of the injectables, by inputting via a section 802 on the interface 800. Further, in some embodiments, the practitioner may also customize the injection planes, the types of injectables and quantity of injectables for every injectable zone independently, e.g., as shown in section 804 of the interface 800 in Fig. 9. In some embodiments, the practitioner may also make adjustments and modifications to the user customized image to facilitate a medically appropriate treatment. The final tentative `after' image may be generated based on the practitioner's inputs and may be displayed on the interface 800 (not shown) and stored in the database 108.

Referring back to Fig. 2, in some implementations, the recommendation system processor 120 further includes a second machine learning module 212 configured to be trained to automatically provide recommendations and facilitate more accurate and enhanced predictions and visualizations of, for example, before and after treatment appearance for the patients. The second machine learning module 212 may be configured to receive the initial image, the system recommended image with injectable zones, user customized images and the final practitioner adjusted images for a number of patients. The second machine learning module 212 is also configured to receive the actual treatment done by the practitioner to validate the predicted recommendations provided by the system 102. For example, when the practitioner uses a different location for injectable zones as compared to the ones provided in the recommendations, then such changes are recorded and used to retrain the second machine learning module 212.

The processing module 206, the recommendation module 208 and the visualization module 210 may utilize the second machine learning module 212 to enhance their respective generated outputs, thereby also generating more accurate predictions of before and after treatment results based on the recommendations for the patients. In some implementations, the actual 'after' image of the patient after completing the final treatment is also stored in the database 108. This actual 'after' image may be used to validate the predicted tentative 'after' image generated by the recommendation system 102 and further retrain the second machine learning module 212 to further enhance the generated tentative 'after' image by the system 102. For example, the second machine learning module 212 may also be iteratively trained until the generated outputs (i.e., the recommendations for injectable zone modifications, the locations of the injectable zones that need adjustments, the before and after visualization of the treatment for the patient, etc.) satisfy a threshold level of accuracy.

In one example, the second machine learning module 212 may utilize a deep learning framework, such as Artificial Neural Network (ANN) and Modular Neural Network (MNN), for training. Further, although the first and second machine learning modules 202, 212 are shown to be implemented as two separate modules, it may be appreciated that in some alternative implementations, they may be combined into a single module as well to achieve the desired functionalities of the present disclosure. Additionally, one or more of the first and second machine learning modules 202, 212 may use supervised learning, such as that described herein, but may also utilize unsupervised learning to achieve similar functionalities.

The recommendation system 102 of the present disclosure provides various image processing and computer vision capabilities using machine learning modules to accurately identify the locations of injectable zones and accurately generate recommendations for augmentations to these injectable zones. Such recommendations are powered by two separate machine learning modules to provide an enhanced accuracy and reliability of the output generated by the system 102. Therefore, these recommendations are less prone to human errors, thereby making these recommendations safe for assisting medical practitioners as well as general practitioners in safely performing these cosmetic procedures with high precision and accuracy.

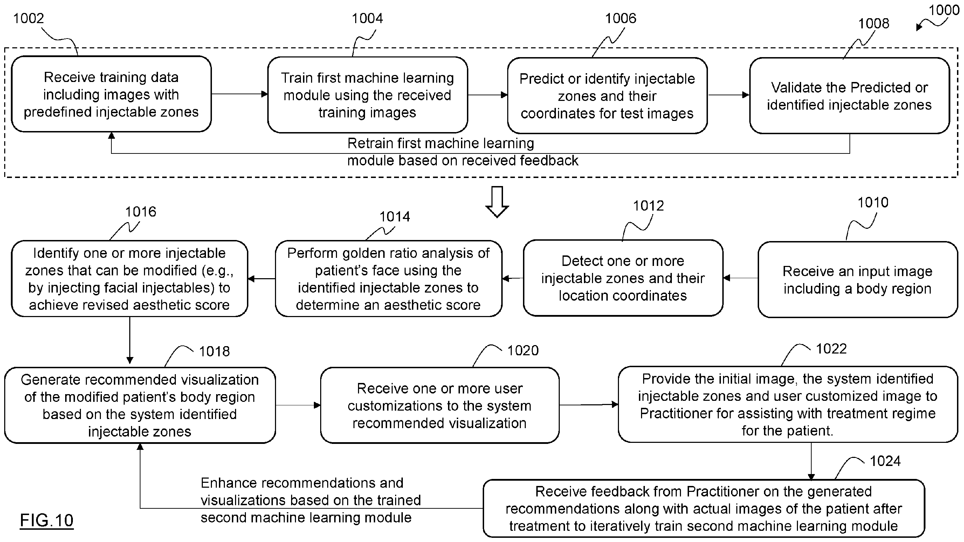

Referring now to Fig. 10, an example method 1000 performed by the injectables recommendation system 102, is illustrated, in accordance with the embodiments of the present disclosure. For example, as explained previously, steps 1002 to 1008 illustrate the steps for training the first machine learning module 202 to process an unmarked patient image to automatically predict or identify injectable zones within a body region present in the image. As the first machine learning module 202 is sufficiently trained, the method may proceed to step 1010, where a new image is received from a patient. At step 1012, the features recognition module 204 may utilize the first machine learning module 202 to automatically predict or identify one or more injectable zones and their location (x/y) coordinates. Further, at step 1014, the body region is analyzed with respect to a predefined criteria, such as the golden ratio, by the processing module 206, to determine an aesthetic score of the body region. Based on the determined aesthetic score, at step 1016, the recommendation module 208 may generate one or more recommendations for one or more injectable zones to be modified, for example, by injecting injectables to obtain a revised aesthetic score that is desired to be close to the golden ratio. Further, the recommended modifications to the injectable zones are then visualized at step 1018, in the form of a visualization, such as an edited image of the patient, to reflect the recommendations made by the system. These visualizations are presented to the patient or the practitioner, such as on the display associated with their respective user devices 104, 106. Further, at step 1020, one or more user customizations provided by the patient (e.g., by making adjustments to the recommendations provided by the system) are received. Once the final customized image is generated to reflect the patient's liking, at step 1022, such image along with the initial image and the identified injectable zones generated by the system 102 are provided to the practitioner to enable them to generate a treatment regime for the patient to achieve the desired look as indicated in the user customized image. The practitioner may validate the recommendations made by the system by either accepting the recommendations or may alternatively change or reject one or more recommendations. Such changes or rejections may be recorded to train a second machine learning module 212 at step 1024. The second machine learning module 212 may be iteratively trained using a number of recommendations generated by the system 102 and the feedback provided by the practitioners for all the recommendations to further enhance the recommendations and visualizations provided by the system, such as those in step 1018.

The system and method according to the embodiments of the present disclosure provide enhanced, efficient, and accurate predictions for performing cosmetic procedures, such as injecting facial injectables to enhance the appearance of any patient. The machine learning based recommendations provide immense support to the practitioners in the domain to accurately assist them in identifying and visualizing the locations of such injectables. In fact, a well-trained system of the present disclosure may be used to even assist and train professionals who may not technically be well experienced in the domain. Additionally, the machine learning based recommendations, being highly accurate, may provide a safer mechanism for guiding the injectables and hence less prone to any undesired complications caused due to human errors.

Further, although the present disclosure is provided with reference to facial injectables, it may be appreciated that these are merely examples and that injectables for enhancing other parts of the body may also be predicted and recommended in a similar manner without limiting the scope. Moreover, other procedures, such as, but not limited to, plastic surgery, dental procedures, skin treatments, may also benefit from the present disclosure, where recommendations for the injectables used in these procedures may also be made by the system and method as described in the present disclosure, in a similar manner.

For simplicity and clarity of illustration, where considered appropriate, reference numerals may be repeated among the figures to indicate corresponding or analogous elements. In addition, numerous specific details are set forth in order to provide a thorough understanding of the examples described herein. However, it will be understood by those of ordinary skill in the art that the examples described herein may be practiced without these specific details. In other instances, well-known methods, procedures, and components have not been described in detail so as not to obscure the examples described herein. Also, the description is not to be considered as limiting the scope of the examples described herein.

It will be appreciated that the examples and corresponding diagrams used herein are for illustrative purposes only. Different configurations and terminology can be used without departing from the principles expressed herein. For instance, components and modules can be added, deleted, modified, or arranged with differing connections without departing from these principles.

The steps or operations in the flow charts and diagrams described herein are just for example. There may be many variations to these steps or operations without departing from the principles discussed above. For instance, the steps may be performed in a differing order, or steps may be added, deleted, or modified. Although the above principles have been described with reference to certain specific examples, various modifications thereof will be apparent to those skilled in the art.

## Claims

1. A method for recommending injectables for a cosmetic treatment, the method comprising:
receiving, by a recommendation system processor, an input image including a body region of a user;
detecting, by the recommendation system processor using a machine learning module, one or more injectable zones within the body region;
determining, by the recommendation system processor, an aesthetic score of the body region based on the detected one or more injectable zones;
identifying, by the recommendation system processor, at least one injectable zone to be modified by injecting an injectable for achieving an augmented body region having a revised aesthetic score that satisfies a predefined threshold; and
generating, by the recommendation system processor, an output recommendation image to be displayed on an output device, the output recommendation image indicating the identified at least one injectable zone to be modified.

2. The method of claim 1, wherein detecting the one or more injectable zones within the body region further comprising detecting, by the recommendation system processor using the machine learning model, location coordinates of each of the one or more injectable zones.

3. The method of claims 1 or 2 further comprising training the machine learning module for detecting the one or more injectable zones within the body region, the training comprising:
receiving, by the machine learning module, training data including a plurality of training images each having predefined injectable zones and one or more parameters associated with the predefined injectable zones, the one or more parameters including a name and a type of each of the plurality of injectable zones;
extracting, by the machine learning module, location coordinates of each of the predefined injectable zones; and
correlating, by the machine learning module, patterns between the predefined injectable zones, the location coordinates of each of the predefined injectable zones, and one or more landmark features of the body region to learn to automatically detect the one or more injectable zones in an unmarked image.

4. The method of claim 3, wherein the training data further includes one or more of type and quantity of injectables suitable for each of the predefined injectable zones and one or more injection planes within the body region for injecting injectables in each of the predefined injectable zones.

5. The method of claim 3, wherein the training further comprising:
comparing, by the recommendation system processor, the detected one or more injectable zones and the location coordinates for each of the one or more injectable zones with real injectable zones and location coordinates provided by a user via a user interface displayed on the output device; and
retraining the machine learning model by providing a feedback based on the comparison.

6. The method of any one of claims 1 to 5, wherein determining the aesthetic score of the body region further comprising:
determining, by the recommendation system processor, one or more feature ratios in the body region within the received input image based on location coordinates of each of the identified one or more injectable zones; and
comparing, by the recommendation system processor, the determined one or more feature ratios with a predefined criteria to determine the aesthetic score of the body region, the aesthetic score being indicative of a degree of match between the one or more feature ratios and the predefined criteria.

7. The method of any one of claims 1 to 6, wherein detecting the one or more injectable zones within the body region further comprising detecting, by the recommendation system processor using the machine learning model, location coordinates of each of the one or more injectable zones and wherein identifying the at least one injectable zone to be modified comprising:
adjusting, by the recommendation system processor, location coordinates of one or more of injectable zones to obtain the revised aesthetic score that satisfies the predefined threshold.

8. The method of any one of claims 1 to 7, further comprising updating, by the recommendation system processor, the identified at least one injectable zone to be modified based on feedback received via a user interface displayed on the output device.

9. The method of any one of claims 1 to 8 further comprising:
receiving, by the recommendation system processor, a user input via a user interface displayed on the output device, the user input including a customization of the at least one identified injectable zone to be modified; and
generating, by the recommendation system processor, a user customized image to be displayed on the output device based on the received user input.

10. The method of claim 9, wherein the user input is received via one or more adjustment tools displayed on the output device.

11. The method of claim 9, wherein the user input includes one or more additional injectable zones to be modified in addition to the identified at least one injectable zone to be modified.

12. The method of any one of claims 1 to 11 further comprising determining, by the recommendation system processor, one or more of an injection plane, a type of injectable, and a quantity of the injectable for the identified at least one injectable zone to be modified, and wherein the generated output recommendation image further includes the determined one or more of the injection plane, the type and quantity of the injectable.

13. The method of any one of claims 1 to 12, wherein the recommendation system processor comprises a second machine learning module and wherein identifying at least one injectable zone to be modified further comprising:
predicting, by recommendation system processor using the second machine learning module, the at least one injectable zone to be modified; and
validating, by the recommendation system processor, the predicted at least one injectable zone based on user feedback.

14. A computer readable medium comprising computer executable instructions for performing the method of any one of claims 1 to 13.

15. A system comprising at least one processor, a memory, and at least one communication interface, the memory comprising computer executable instructions for causing the processor to perform the method of any one of claims 1 to 13.
